# FASCICULE DE BREVET EUROPEEN

(11) **EP 3 934 722 B1**
(45) Date de publication et mention de la délivrance du brevet: **07.06.2023**
(21) Numéro de dépôt: 20725846.8
(22) Date de dépôt: 06.03.2020
(51) Int. Cl.: A61M 16/00, F21S 10/00, F21V 33/00, H05B 47/105

(54) **DISPOSITIF LUMINEUX DE VENTILATION RESPIRATOIRE**
LEUCHTENDE LUNGENVENTILATIONSVORRICHTUNG
LUMINOUS RESPIRATORY VENTILATION DEVICE

(30) Priorité: 08.03.2019 FR 1902418
(43) Date de publication de la demande: 12.01.2022
(60) Demande divisionnaire: 22203695.6
(73) Titulaire: Sleepinnov Technology, 38430 Moirans (FR)
(72) Inventeur: HUNGR, Nikolai, 38430 MOIRANS (FR); ARGOD, Jérôme, 38430 MOIRANS (FR); LANTELME, Marc, 38430 MOIRANS (FR); GAIFFE, Nicolas, 38430 MOIRANS (FR)
(74) Mandataire: INNOV-GROUP
(86) Numéro de dépôt international: PCT/FR2020/050465
(87) Numéro de publication internationale: WO 2020/183097

(56) Documents cités:
- EP-A1- 2 371 411
- WO-A1-2012/106775
- CN-A- 107 929 895
- US-A1- 2005 235 993
- US-A1- 2007 023 044
- US-A1- 2007 193 582
- US-A1- 2017 119 919
- US-B2- 8 453 640

## Description

### DOMAINE TECHNIQUE

Le domaine technique de l'invention est un dispositif de ventilation par pression positive continue. Ce type de dispositif est couramment utilisé dans le traitement de l'apnée du sommeil.

### ART ANTERIEUR

Le recours à la ventilation par pression positive continue (PPC) constitue un traitement de référence dans le domaine de l'apnée du sommeil. Ce traitement consiste à insuffler de l'air de façon continue dans un masque appliqué sur le visage d'un utilisateur. Il peut s'agir d'un masque nasal, narinaire ou facial. Le souffle d'air parvient aux voies respiratoires de l'utilisateur, en exerçant une pression suffisante sur ces dernières, de façon à prévenir la formation d'un collapsus.

Ces dispositifs de ventilation positive par PPC sont utilisés la nuit. Par conséquent, ils doivent être le plus silencieux possible. Parmi les dispositifs de l'art antérieur, citons par exemple le dispositif décrit dans US8453640. Un tel dispositif est portable et présente une forme sensiblement cylindrique, s'étendant à partir d'une base plane.

Le document EP2371411 décrit un dispositif de type PPC comportant une ou plusieurs source de lumière, disposées sur un tube débouchant sur un masque ou sur le boîtier du dispositif. Le document WO2012106775 décrit un dispositif de type PPC comportant des sources de lumière pouvant être modulées en fonction d'un débit d'air. Les sources de lumières sont disposées soit sur le masque, soit sur un boîtier. Le document US2018126104 décrit un dispositif de ventilation permettant de détecteur une survenue d'une toux, et déclenchant alors une alarme. Ce dispositif comporte une source de lumière disposée sur un boîtier.

D'autres types de dispositifs de ventilation sont décrits dans WO2011006199, US20050235993, US20070023044, US20070193582 ou US20110308518.

Les inventeurs ont conçu un dispositif de respiration ventilatoire, présentant des fonctionnalités supplémentaires par rapport aux dispositifs actuellement disponibles.

### EXPOSE DE L'INVENTION

Un premier objet de l'invention est un dispositif de ventilation respiratoire, destiné à envoyer un flux d'air, généré par un ventilateur, dans un conduit, le conduit s'étendant entre le dispositif et un masque respiratoire destiné à être porté par un utilisateur, le dispositif comportant :
- une entrée d'air, destinée à admettre l'air dans le dispositif;
- un ventilateur;
- une sortie d'air, la sortie d'air étant configurée pour être raccordée au conduit;
   de telle sorte que lorsque le ventilateur fonctionne, l'air s'écoule, à travers le dispositif depuis l'entrée d'air successivement vers le ventilateur puis vers la sortie d'air ;
   le dispositif comportant également :
- un capteur de débit, mesurant un débit d'air circulant à travers le dispositif, et/ou un capteur de pression mesurant une pression entre le ventilateur et la sortie d'air;
- une unité de commande, reliée au capteur de débit et/ou au capteur de pression;
- une enceinte, définissant l'entrée d'air et la sortie d'air, l'enceinte renfermant le ventilateur;

- l'enceinte s'étendant, autour d'un axe longitudinal, entre une base et un sommet;
- l'enceinte comportant une paroi latérale reliant la base et le sommet, la paroi latérale s'étendant autour de l'axe longitudinal;
le dispositif étant caractérisé en ce que:
- l'enceinte comprenant au moins un support, formant une courbe, et s'étendant à l'intérieur de l'enceinte, autour de l'axe longitudinal, le support étant disposé en regard de la paroi latérale, le support portant une pluralité de sources de lumière, chaque source de lumière étant configurée pour émettre une lumière;
- l'enceinte étant formée d'un matériau translucide et/ou diffusant;
de telle sorte que sous l'effet d'une activation des sources de lumière fixées au support, la lumière, produite par les sources de lumière, diffuse à travers la paroi latérale de l'enceinte. Selon un mode de réalisation, le dispositif comporte l'une des caractéristiques suivantes, prises isolément ou selon les combinaisons techniquement réalisables.
- Le dispositif comporte au moins une source de lumière, apte à émettre une lumière se propageant à l'extérieur du dispositif, la source de lumière étant pilotée par l'unité de commande, de telle sorte que l'unité de commande est configurée pour activer la source de lumière en fonction du débit d'air mesuré par le capteur de débit et/ou de la pression mesurée par le capteur de pression. Par pilotée, il est entendu qu'un pilotage de l'intensité de la source de lumière et/ou un pilotage de la couleur de la lumière émise.
- L'unité de commande est configurée pour moduler une couleur de la lumière émise par la source de lumière, ou par chaque source de lumière.
- L'unité de commande est configurée pour moduler une intensité lumineuse et/ou une couleur de la source de lumière, ou de chaque source de lumière, selon une période de modulation, la période de modulation comportant :
   ▪ une augmentation progressive de l'intensité lumineuse suivie d'une diminution progressive de l'intensité lumineuse ;
   ▪ et/ou une variation progressive de la couleur entre une couleur de départ et une couleur d'arrivée, suivie d'une variation progressive de la couleur de la lumière émise entre la couleur d'arrivée et la couleur de départ.
- La période de modulation peut être réglable.
- L'unité de commande est configurée pour:
   ▪ estimer une fréquence respiratoire en fonction des débits mesurés par le capteur de débit;
   ▪ déterminer la période de modulation en fonction de la fréquence respiratoire estimée.
- L'unité de commande est configurée pour:
   ▪ estimer un rythme cardiaque en fonction des débits mesurés par le capteur de débit;
   ▪ déterminer la période de modulation en fonction du rythme cardiaque estimé.
- L'unité de commande est configurée pour activer la source de lumière, ou chaque source de lumière, selon une séquence d'alerte, lorsque le débit d'air est en dehors d'une plage de débit prédéterminée, ou lorsque le débit d'air est supérieur ou inférieur à une valeur seuil de débit prédéfinie.
- L'unité de commande est configurée pour déterminer une variation temporelle des débits mesurés par le capteur de débit, en différents instants successifs, et pour activer la source de lumière, ou chaque source de lumière, selon une séquence d'alerte, lorsque la variation temporelle est inférieure à un seuil de variation, ce dernier étant de préférence prédéfini.
- L'unité de commande est configurée pour activer la source de lumière, selon une séquence d'alerte, lorsque la pression d'air est en dehors d'une plage de pression prédéterminée, ou est inférieure ou inférieure à une valeur seuil de pression prédéfinie.
- La paroi latérale s'étend autour de l'axe longitudinal selon un premier rayon.
- L'enceinte renferme un réflecteur de lumière, le réflecteur s'étendant autour de l'axe longitudinal, selon un deuxième rayon inférieur au premier rayon, de telle sorte que le réflecteur est apte à réfléchir une lumière produite par les sources de lumière vers l'enceinte.
- Les sources de lumière sont configurées pour émettre une lumière en direction du sommet de l'enceinte.
- Au moins une source de lumière, voire chaque source de lumière, émet une lumière selon un cône d'émission s'étendant autour d'un axe d'émission, l'axe d'émission de la source de lumière, ou de chaque source de lumière, étant orienté en direction du sommet de l'enceinte.
- Les sources de lumière sont espacées autour de l'axe longitudinal.
- Au moins une source de lumière, voire chaque source de lumière, s'étend entre le support et l'axe longitudinal, de telle sorte que le support est interposé entre au moins une source de lumière, voire chaque source de lumière, et l'enceinte.
- L'enceinte comporte une horloge, reliée à l'unité de commande, de telle sorte que l'unité de commande est configurée pour moduler une intensité lumineuse et/ou la couleur de la lumière des sources de lumière en fonction de l'horloge.

Un deuxième objet de l'invention est un procédé de commande d'une source de lumière d'un dispositif selon le premier objet de l'invention, comportant les étapes suivantes :
a) mesure de débits à l'aide du capteur de débit et/ou de pressions à l'aide du capteur de pression respectivement à différents instants de mesure ;
b) en fonction des valeurs de débit et/ou de pression mesurés, activation de la source de lumière par l'unité de commande.

Selon un mode de réalisation, lors de l'étape b), l'activation de la source de lumière est configurée pour moduler une intensité lumineuse et/ou une couleur de la source de lumière selon une période de modulation, la période de modulation comportant une augmentation progressive de l'intensité lumineuse suivie d'une diminution progressive de l'intensité lumineuse et/ou une variation progressive de la couleur entre une couleur de départ et une couleur d'arrivée, suivie d'une variation progressive de la couleur de la lumière émise entre la couleur de d'arrivée et la couleur de départ.

Selon un mode de réalisation, l'étape a) comporte une mesure de débits à différents instants de mesure, ainsi qu'une estimation d'une fréquence respiratoire en fonction des débits mesurés, de telle sorte que lors de l'étape b), la période de modulation est déterminée en fonction de la fréquence respiratoire estimée.

Selon un mode de réalisation, l'étape a) comporte une mesure de débits à différents instants de mesure, ainsi qu'une estimation d'un rythme cardiaque en fonction des débits mesurés, de telle sorte que lors de l'étape b), la période de modulation est déterminée en fonction du rythme cardiaque estimé.

Selon un mode de réalisation, l'étape b) comporte une activation de la source de lumière selon une séquence d'alerte lorsque :
- un débit d'air mesuré lors de l'étape a) est supérieure à une valeur seuil de débit prédéfinie ;
- et/ou une variation du débit d'air, durant une plage temporelle déterminée, est inférieure à un seuil de variation ;
- et/ou une pression d'air mesurée lors de l'étape a) est inférieure à une valeur seuil de pression prédéfinie.

Selon un mode de réalisation, le dispositif comporte un capteur de débit de l'air entrant ou sortant du dispositif, l'unité de commande étant reliée au capteur de débit, de telle sorte que l'unité de commande est configurée pour moduler une intensité lumineuse des sources de lumière en fonction du débit mesuré par le capteur de débit.

L'unité de commande peut être configurée pour :
- déterminer une fréquence respiratoire en fonction des mesures du capteur de débit;
- déterminer la période de modulation en fonction du rythme respiratoire.

Selon un mode de réalisation, le dispositif comporte un capteur de pression de l'air insufflé par le dispositif, l'unité de commande étant reliée au capteur de pression de telle sorte que l'unité de commande est configurée pour activer les sources de lumière en fonction de la pression mesurée par le capteur de pression.

D'autres avantages et caractéristiques ressortiront plus clairement de la description qui va suivre de modes particuliers de réalisation de l'invention, donnés à titre d'exemples non limitatifs, et représentés sur les figures listées ci-dessous.

### FIGURES

La figure 1A est un schéma montrant une utilisation du dispositif.
Les figures 1B et 1C sont des vues de l'enceinte du dispositif.
La figure 1D montre une partie de l'enceinte du dispositif, à laquelle on a retiré un volet amovible.
La figure 1E montre des composants du dispositif situés à l'intérieur de l'enceinte.
La figure 2A montre un support sur lequel sont fixées des sources de lumière.
Les figures 2B et 2C montrent un réflecteur autour duquel est disposé le support.
La figure 2D est une vue de dessus, montrant l'agencement des sources de lumière, portées par le support, autour du réflecteur.
La figure 3 montre l'enceinte, représentée en transparence, à l'intérieur de laquelle s'étend le réflecteur ainsi que le support des sources de lumière.
La figure 4 est une vue de l'extérieur de l'enceinte.
La figure 5A schématise une mesure d'un débit d'air résultant d'un débitmètre disposé dans le dispositif.
La figure 5B schématise une modulation de l'intensité lumineuse des sources de lumière.
Les figures 6A et 6B schématisent des enchaînements d'étapes mettant en oeuvre des mesures de débits ou de pression.

### EXPOSE DE MODES DE REALISATION PARTICULIERS

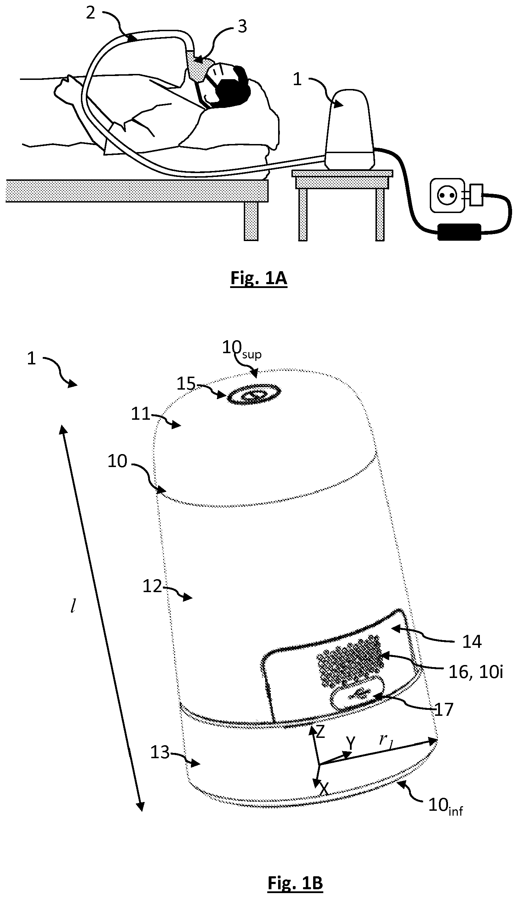

La figure 1A montre un dispositif 1 d'aide à la respiration selon l'invention. Le dispositif comporte un conduit 2, le reliant à un masque respiratoire 3 destiné à être appliqué sur le visage d'un utilisateur. De préférence, le conduit 2 est un conduit souple, dont la longueur est de quelques mètres. Le dispositif 1 comporte un ventilateur, commandé pour maintenir une pression de consigne au niveau du masque respiratoire. Il est essentiellement destiné à un usage nocturne. Lors de son utilisation, le dispositif peut notamment être disposé sur un support plan, par exemple une table de chevet.

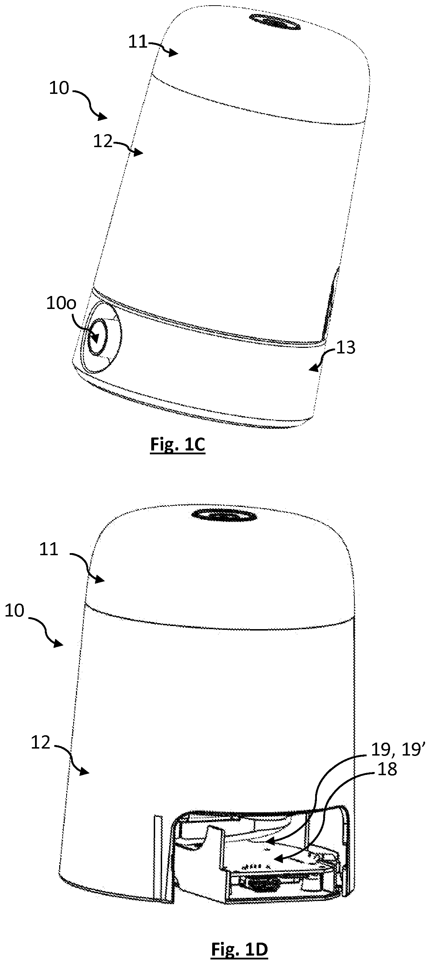

Les figures 1B et 1C montrent une enceinte 10 du dispositif 1. L'enceinte 10 s'étend selon un axe longitudinal Z, en définissant une longueur l, entre une base 10_{inf} et un sommet 10ₛᵤₚ. L'enceinte s'étend radialement selon un plan de base XY, dans lequel elle définit un premier rayon r₁. La longueur *l* est typiquement comprise entre 10 cm et 40 cm, tandis que le premier rayon r₁ est généralement compris entre 5 cm et 30 cm. Les dimensions du dispositif le rendent aisément transportable. Par ailleurs, le dispositif est adapté à être déposé sur un support plan de faibles dimensions, par exemple une table. Lors de l'utilisation, l'axe longitudinal Z est de préférence parallèle à la verticale, tandis que le plan de base XY est horizontal.

L'enceinte 10 est de préférence constituée d'un matériau rigide, par exemple un plastique. Le matériau constituant l'enceinte n'est pas opaque. Il s'agit d'un matériau de préférence translucide et/ou diffusant à travers lequel la lumière diffuse, sans pour autant être transparent : ainsi, il n'est pas possible, depuis l'extérieur de l'enceinte, d'identifier les composants disposés à l'intérieur de cette dernière. L'enceinte peut être transparente, mais cela ne correspond pas au mode de réalisation préféré. Dans l'exemple représenté, l'enceinte 10 comporte une paroi latérale, définissant un tronçon supérieur 11, un tronçon intermédiaire 12 et un tronçon inférieur 13. Le tronçon inférieur 13 est délimité par une surface inférieure, formant la base 10_{inf} de l'enceinte. La base de l'enceinte 10_{inf} de préférence plane et parallèle au plan de base XY. La base de l'enceinte 10_{inf} est destinée à être disposée sur un support plan.

Le tronçon intermédiaire 12 comporte une grille 16, agissant en tant que filtre, et formant une entrée d'air 10i, à travers laquelle l'air peut pénétrer à l'intérieur de l'enceinte 10. Le tronçon intermédiaire 12 comporte également une embase de connexion 17, permettant un raccordement du dispositif 1 à une unité de traitement distante, de façon à paramétrer une unité de commande 18 disposée à l'intérieur de l'enceinte 10 et décrite en lien avec la figure 1D.

Dans cet exemple, l'embase de connexion 17 et l'entrée d'air 10i sont disposées sous un volet amovible 14.

Sur le tronçon supérieur 11 est disposé un commutateur de commande marche/arrêt 15. La disposition au sommet de l'enceinte 10 le rend aisément accessible par un utilisateur, y compris dans la pénombre ou l'obscurité. Le commutateur de commande 15 peut comporter une source de lumière, par exemple une diode électroluminescente, de façon à être visible dans l'obscurité. L'enceinte 10 est de préférence symétrique autour de l'axe longitudinal Z. Le commutateur de commande marche/arrêt est alors centré autour de l'axe longitudinal Z. Ainsi, quelle que soit la rotation du dispositif 1, autour de l'axe longitudinal Z, le commutateur marche/arrêt 15 ne bouge pas : sa position est indépendante de la rotation du dispositif Z autour de l'axe longitudinal Z.

Sur la figure 1C, on observe que le tronçon inférieur 13 comporte une ouverture de sortie 10o, formant une sortie d'air du dispositif 1. L'ouverture de sortie 10o est destinée à être raccordée au conduit 2, représenté sur la figure 1A, lors de l'utilisation du dispositif 1.

La figure 1D montre le tronçon intermédiaire 12 et le tronçon supérieur 11. Sur la figure 1D, on observe une unité de commande 18, permettant le paramétrage et/ou le pilotage du dispositif 1, et en particulier la commande du ventilateur. L'unité de commande 18 peut être paramétrée par une liaison filaire raccordée à l'embase 17, comme précédemment décrit. L'unité de commande 18 prend la forme d'une carte électronique de type PCB (Printed Circuit Board).

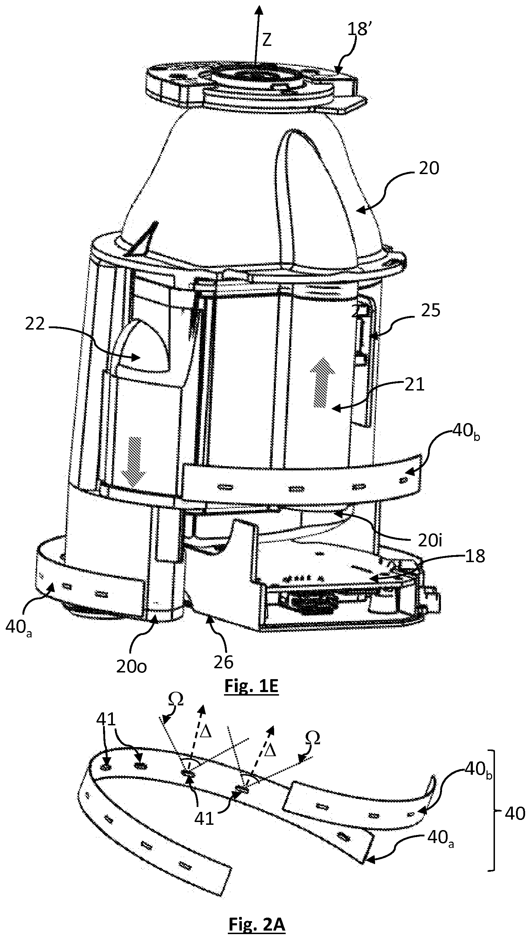

La figure 1E représente l'intérieur du tronçon intermédiaire 12 et du tronçon supérieur 11. L'air admis dans l'enceinte 10, à travers l'ouverture 10i, s'écoule à travers un tube d'entrée 21, en direction du sommet du dispositif, parallèlement ou sensiblement parallèlement à l'axe longitudinal Z. L'air est ensuite admis dans une coque 20, dans laquelle s'étend une chambre de ventilation, comportant le ventilateur. Sur la figure 1E, le ventilateur est masqué par la coque 20. Le ventilateur permet d'aspirer l'air de l'ouverture 10i jusqu'à la chambre de ventilation. Le ventilateur génère un flux d'air se propageant à travers un tube de sortie 22. Le tube de sortie débouche dans le tronçon inférieur 13, par une ouverture 20o. L'ouverture 20o est ménagée au niveau de l'interface entre le tronçon intermédiaire 12 et le tronçon inférieur 13. L'air, débouchant dans le tronçon inférieur 13 par l'ouverture 20o, s'écoule, dans le tronçon inférieur 13, vers l'ouverture de sortie 10o.

Le dispositif comporte également une carte électronique auxiliaire 18', reliée au commutateur de mise en service 15. La carte électronique auxiliaire 18' est également reliée à l'unité de commande 18.

Le dispositif 1 comporte un débitmètre 25, relié à l'unité de commande 18. Le débitmètre 25 permet une mesure du débit d'air se propageant dans le tube d'entrée 21, sous l'effet de l'aspiration par le ventilateur. Dans cet exemple, le débitmètre 25 est ménagé au niveau du tube d'entrée 21. Le débitmètre 25 permet d'ajuster une pression de consigne de l'air adressé par le dispositif 1 vers le masque 3 de l'utilisateur. Par exemple, lorsque l'utilisateur est victime d'une apnée du sommeil, le débit d'air chute, ce qui entraîne une augmentation de la pression de consigne.

Le dispositif peut comporter un capteur de pression 26, mesurant la pression de l'air entre le ventilateur et la sortie d'air 1o. En fonction de la pression mesurée, une unité de commande adapte la puissance du ventilateur de façon à maintenir une pression aussi stable que possible autour de la pression de consigne.

Le dispositif est destiné à être utilisé dans l'obscurité. Afin d'être visible, le dispositif comporte des sources de lumière 41 disposées sur au moins un support 40. Dans l'exemple représenté, le support 40 décrit une courbe : le support 40 est annulaire. Par support annulaire, on entend un support décrivant tout ou partie d'un anneau, autour de l'axe longitudinal Z. Dans l'exemple représenté sur la figure 2A, le support annulaire 40 comporte un premier support 40ₐ, et un deuxième support 40_{b}, le premier support et le deuxième support étant disjoints, de façon à se conformer à la géométrie du dispositif.

La figure 1E montre la disposition du premier support 40ₐ, au niveau de la partie inférieure du tronçon intermédiaire 12, ainsi que la disposition du deuxième support 40_{b}, en surplomb de l'unité de commande 18. Les sources de lumière 41 sont reliées à un circuit d'activation 19, faisant partie de l'unité de commande 18, permettant leur activation et une modulation éventuelle de leur intensité.

Chaque support annulaire comporte au moins une source de lumière 41. Chaque source de lumière 41 est par exemple une diode électroluminescente. De préférence, les sources de lumière sont régulièrement espacées sur un support annulaire. De préférence, le support 40 est agencé de telle sorte que les sources de lumière 41 sont réparties, de préférence régulièrement, autour de l'axe longitudinal Z. Dans l'exemple représenté, le premier support 40ₐ comporte 11 sources de lumière 41 régulièrement espacées. Le deuxième support 40_{b} comporte 4 sources de lumière 41 régulièrement espacées. Le fait de disposer plusieurs sources de lumière sur un même support rend la fabrication plus simple et moins onéreuse. Dans la suite de la description, le support 40 désigne l'ensemble formé par le premier support 40ₐ et le deuxième support 40_{b}.. Le support 40 peut être souple, ce qui est moins onéreux à fabriquer.

Le support 40 définit une courbe. Chaque source de lumière est orientée à l'intérieur d'un espace convexe délimité par la courbe. Ainsi, chaque source de lumière est disposée à l'intérieur d'un espace convexe délimité par le support 40, entre ce dernier et l'axe longitudinal Z. Ainsi, le support 40 est interposé entre l'enceinte 10 et les sources de lumière. Le support 40 est avantageusement opaque. Il forme alors un bandeau opaque interposé entre la partie lumineuse de chaque source de lumière 41 et l'enceinte 10. Ainsi, la lumière émise par chaque source de lumière atteint l'enceinte 10 indirectement, en étant réfléchie ou rétrodiffusée, comme expliqué par la suite. La hauteur du support, parallèlement à l'axe longitudinal Z, est de préférence comprise entre 1 et 5 cm, de préférence entre 1 et 3 cm.

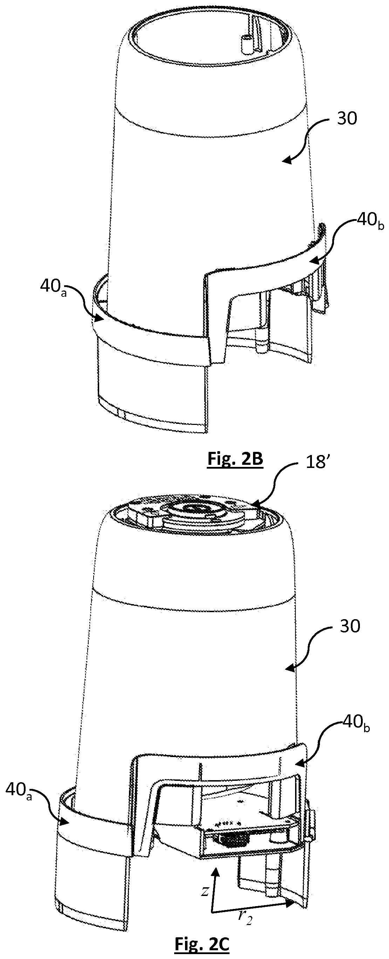

De préférence, chaque source de lumière 41 génère une lumière selon un cône d'émission Ω s'étendant autour d'un axe d'émission Δ. L'axe d'émission Δ associé à chaque source de lumière 41 est parallèlement, ou sensiblement parallèlement, à l'axe longitudinal Z, vers le tronçon supérieur 11 de l'enceinte 10. Ainsi, la lumière émise par chaque source de lumière 41 se propage, lors de l'émission, vers le tronçon supérieur 11 (ou vers le sommet 10ₛᵤₚ de l'enceinte 10). Dans l'exemple représenté, les sources de lumière sont des diodes electroluminescentes dites "side illuminating", ce qui signifie éclairant sur le côté. Du fait de l'ouverture du cône d'émission Ω, la lumière émise par chaque source de lumière 41 est rétrodiffusée soit par le support 40 (en l'occurrence le premier support 40ₐ ou le deuxième support 40_{b}), soit par un réflecteur 30 décrit en lien avec les figures 2B et 2C. Sur la figure 2A, on a représenté deux cônes d'émission Ω respectivement associés à deux sources de lumière 41, et centrés autour d'axes d'émission Δ orientés vers le tronçon supérieur 11 de l'enceinte 10.

Les figures 2B et 2C représentent le réflecteur 30, diffusant ou opaque, recouvrant la coque 20 représentée sur la figure 1E, ainsi que les tubes d'arrivée 21 et de sortie 22. Le réflecteur 30 est entouré par le support 40. Dans l'exemple représenté, le réflecteur 30 s'étend selon une géométrie tubulaire, selon un deuxième rayon *r₂* strictement inférieur au premier rayon *r₁* de l'enceinte 10. Le réflecteur 30 est dimensionné pour permettre que le support 40 soit interposé entre l'enceinte 10 et le réflecteur 30. Le réflecteur 30 s'étend face aux sources de lumière 41 portées par le support 40. La fonction du réflecteur 30 est de réfléchir, ou de rétrodiffuser, la lumière émise par chaque source de lumière 41 vers l'enceinte 10. Du fait de l'agencement du support 40, des sources de lumière 41 et du réflecteur 30, le support 40 forme un masque opaque empêchant la lumière émise par chaque source de lumière 41 d'atteindre directement l'enceinte 10, avant d'être réfléchie ou rétrodiffusée par le réflecteur 30. Ainsi, la majeure partie de la lumière, émise par les sources de lumière 41, et atteignant l'enceinte 10, est réfléchie ou rétrodiffusée par le réflecteur 30. La lumière 41 atteint ainsi l'enceinte 10 de façon indirecte. Une partie de la lumière atteignant l'enceinte 10 diffuse à travers cette dernière vers l'extérieur de l'enceinte. Une autre partie de la lumière atteignant l'enceinte est rétrodiffusée par cette dernière vers le réflecteur 30. Ainsi, vue de l'extérieur, l'enceinte forme une source de lumière secondaire, générant un éclairage diffus. Il en résulte un dispositif d'apparence agréable, sans présence de points chauds, c'est-à-dire sans inhomogénéité locale de l'éclairage, susceptible de gêner l'utilisateur. A l'intérieur de l'enceinte, la lumière se propage entre le réflecteur 30 et l'enceinte 10, en direction du sommet 10ₛᵤₚ de l'enceinte.

La hauteur du réflecteur, parallèlement à l'axe longitudinal Z, est comprise entre la hauteur du support et celle de l'enceinte. De préférence, elle correspond à au moins 50% de la hauteur de l'enceinte. De préférence, la hauteur du support 30 est au moins trois ou quatre fois supérieure à la hauteur du support 40.

Du fait de l'agencement décrit ci-dessus, les sources de lumière 41, le support 40 et le réflecteur 30 ne sont pas perceptibles depuis l'extérieur de l'enceinte. L'éclairage secondaire produit par l'enceinte, formé par la lumière diffusant à travers le tronçon intermédiaire 12 de l'enceinte 10, forme un dégradé, l'intensité diffusée se réduisant au fur et à mesure que l'on se rapproche du tronçon supérieur 13. Par source de lumière secondaire ou éclairage secondaire, il est entendu que la lumière n'est pas émise par l'enceinte, mais simplement diffusée par cette dernière, vers l'extérieur de l'enceinte 10, et en particulier vers l'utilisateur.

Du fait de la présence d'éléments opaques disposés entre le support 40 et le tronçon inférieur 13 de l'enceinte, pas ou peu de lumière atteint le tronçon inférieur de l'enceinte.

Sur la figure 2C, on a représenté la carte auxiliaire 18', disposée à l'intérieur de l'enceinte 10, au niveau du tronçon supérieur 13.

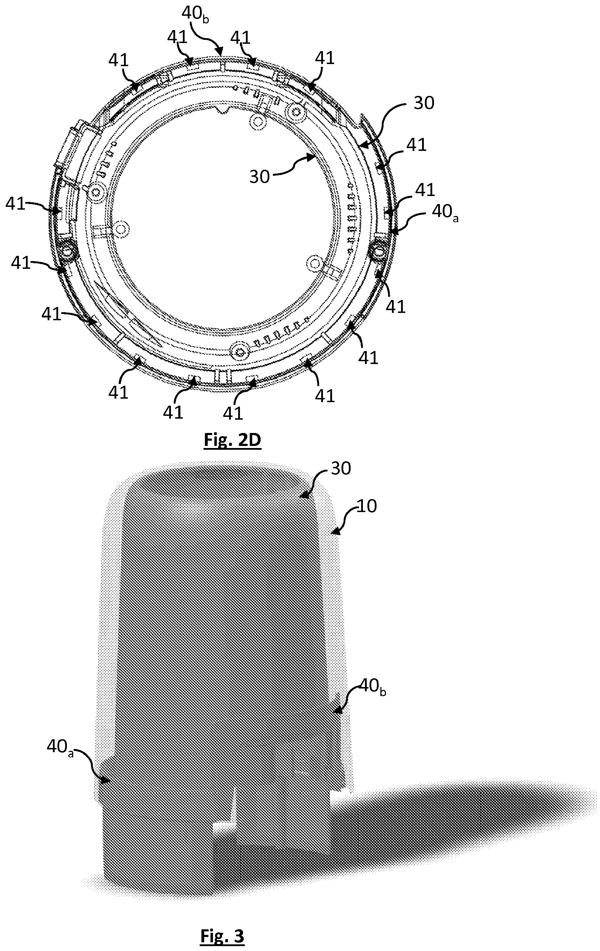

La figure 2D est une vue de dessus du réflecteur 30 et du support 40 (premier support 40ₐ et deuxième support 40_{b}) portant les sources de lumière 41. On observe la répartition des sources de lumière 41 autour du réflecteur 30, dans un espace compris entre le support 40 et le réflecteur 30.

La figure 3 montre l'enceinte 10, en transparence, laquelle se superpose au réflecteur 30. Comme précédemment décrit, l'enceinte 10 forme un diffuseur de la lumière réfléchie ou rétrodiffusée par le réflecteur 30. Elle permet une diffusion de la lumière vers l'extérieur de l'enceinte, formant ainsi une source de lumière secondaire.

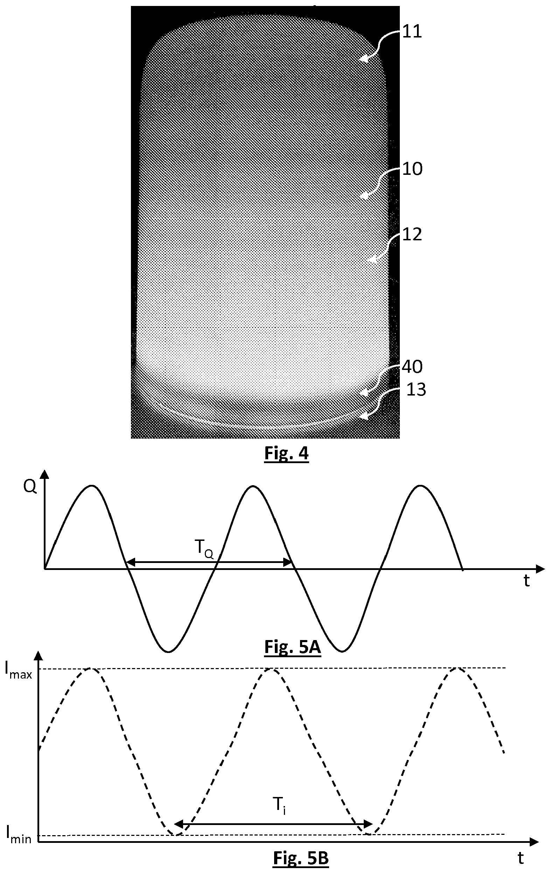

La figure 4 est une photographie d'un exemple d'enceinte 10 selon l'invention. On distingue les tronçons supérieur 11, intermédiaire 12 et inférieur 13 de l'enceinte 10, ainsi que le support 40. On observe que le dispositif permet d'obtenir un dégradé de l'intensité lumineuse.

La lumière émise par le dispositif 1 peut avoir plusieurs fonctions, ce qui est décrit ci-après.

Elle peut faire office de lampe assurant un éclairage, de type lampe de chevet. Elle peut également être raccordée à une horloge 19', située dans l'enceinte, et par exemple au niveau de l'unité de commande 18. Dans l'exemple représenté, l'horloge est disposée sur l'unité de commande 18. En fonction de l'heure indiquée par l'horloge 19', l'unité de commande peut activer progressivement les sources de lumière 41, en modulant l'intensité lumineuse émise. On retrouve alors un fonctionnement similaire à un simulateur d'aube.

Selon un mode de réalisation, schématisé sur les figures 5A et 5B, le dispositif permet une modulation de l'intensité lumineuse des sources de lumière 41, selon une période d'illumination régulière Tᵢ, de telle sorte que l'utilisateur puisse accorder l'expiration et l'inspiration en fonction de la fréquence de modulation. La figure 5A montre une évolution temporelle du débit d'air Q mesuré par le capteur de débit. La figure 5B illustre une évolution de l'intensité des sources lumineuses. Ainsi, le dispositif forme un métronome définissant une fréquence respiratoire prédéterminée T_{Q}, et éventuellement réglable, par exemple manuellement. Par exemple, chaque période de modulation Tᵢ comporte une phase d'intensité lumineuse progressivement croissante, passant d'une intensité minimale Iₘᵢₙ à une intensité maximale Iₘₐₓ, suivie d'une phase d'intensité lumineuse progressivement décroissante, passant de l'intensité maximale Iₘₐₓ à l'intensité minimale Iₘᵢₙ. La période de modulation Tᵢ peut être typiquement être comprise entre 3s et 15s. Il est généralement admis que la fréquence respiratoire d'un utilisateur est voisine de 10s, soit 6 respirations par minute. Par exemple, au cours d'une demi-période, l'utilisateur inspire (ou expire) sous l'effet de l'augmentation de l'intensité lumineuse. Au cours de l'autre demi-période, l'utilisateur expire (ou inspire), sous l'effet de la diminution de l'intensité lumineuse.

De façon alternative ou complémentaire, au cours de la période de modulation, la couleur d'une ou de chaque source de lumière évolue progressivement entre une couleur de départ (par exemple le bleu) et une couleur d'arrivée (par exemple le rouge), puis de la couleur d'arrivée vers la couleur de départ.

Au cours d'une même période d'illumination Tᵢ, les phases d'augmentation et de diminution de l'intensité lumineuse peuvent être de même durée, ou être de durées différentes, l'augmentation étant par exemple plus longue que la diminution ou réciproquement. En fonction de la modulation de l'intensité lumineuse, l'utilisateur peut adapter sa respiration, ce qui permet un effet relaxant, selon les principes de la cohérence cardiaque. Cela peut constituer une aide à l'endormissement. La modulation de l'intensité et/ou de la couleur des sources de lumière peut être réglable par le patient.

Selon un mode de réalisation, la modulation de chaque source de lumière est commandée par le capteur de débit 25. En effet, la mesure donnée par le capteur de débit 25 permet une estimation d'une fréquence respiratoire T_{Q} de l'utilisateur. En fonction de la fréquence respiratoire T_{Q} de l'utilisateur, la période de modulation Tᵢ peut être définie, en particulier pour progressivement ralentir la fréquence respiratoire. Ainsi, en fonction des mesures délivrées par le capteur de débit 25, l'unité de commande 18 détermine une période de modulation Tᵢ de l'intensité lumineuse plus lente que la fréquence respiratoire T_{Q}, jusqu'à ce que le rythme respiratoire T_{Q} mesuré par le capteur de débit se situe dans une plage acceptable prédéterminée.

Selon un autre mode de réalisation, à partir du débit mesuré par le débitmètre, il est possible d'estimer un rythme cardiaque HR de l'utilisateur. La période de modulation est alors ajustée, par l'unité de commande 18, de façon à adapter la période de modulation Tᵢ des sources de lumière en fonction du rythme cardiaque. La période de modulation correspond de préférence à une fréquence respiratoire adaptée au rythme cardiaque.

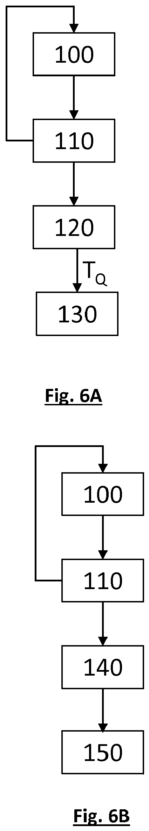

La figure 6A résume les principales étapes des modes de réalisation précédemment décrits :
Etape 100 : mesure du débit d'air par le capteur de débit à un instant de mesure ;
Etape 110 : réitération de l'étape 100 ;
Etape 120 : à partir de plusieurs mesures de débit d'air à différents instants de mesure, estimation d'une fréquence respiratoire T_{Q}, et éventuellement d'un rythme cardiaque HR ;
Etape 130 : adaptation de la période de modulation Tᵢ en fonction des estimations de la fréquence respiratoire T_{Q} ou du rythme cardiaque HR.

Selon un mode de réalisation, le capteur de débit 25 est relié à l'unité de commande 18. En cas de retrait intempestif du masque, la pression mesurée par le capteur de pression 26 tend à diminuer, ce qui entraîne une augmentation du débit du ventilateur de façon que la pression mesurée corresponde à la pression de consigne. Ainsi, le débit d'air, mesuré par le capteur de débit 25, augmente. Au-delà d'une valeur seuil de débit, le dispositif se met en alerte. L'unité de commande active les sources de lumière selon une séquence d'alerte, par exemple en modifiant la couleur (ou l'intensité) de la lumière émise, la lumière pouvant par exemple être rouge. La valeur seuil de débit peut être préalablement définie. Lorsque le débit d'air, mesuré par le capteur de débit 25, est normal, la lumière émise par les sources de lumière peut être bleue ou blanche. Le terme séquence d'alerte désigne une activation de la source de lumière représentative d'une alerte, par exemple une lumière rouge et/ou clignotement rapide...

Selon un mode de réalisation, le capteur de pression 26 est relié à l'unité de commande 18. En cas de retrait intempestif du masque, la pression mesurée par le capteur de pression 26 peut ne plus suivre la pression de consigne, et chuter en dessous de la pression de consigne. En cas de chute de la pression mesurée en dessous d'une valeur seuil de pression, susceptible de correspondre à un retrait intempestif du masque 3, l'unité de commande 18 active les sources de lumière 41, selon la séquence d'alerte. La pression seuil peut être préalablement définie.

D'une manière plus générale, on peut définir une plage de débit acceptable, et activer les sources de lumière selon la séquence d'alerte lorsque le débit mesuré par le capteur de débit 25 sort de la plage de débit. De façon analogue, on peut définir une plage de pression acceptable, et activer les sources de lumière selon la séquence d'alerte, lorsque la pression mesurée par le capteur de pression 26 sort de la plage de pression.

Selon un autre mode de réalisation, un retrait du masque peut être détecté par le capteur de débit, en particulier en analysant des variations temporelles du débit mesuré durant une plage temporelle. Lorsque le débit est stable, cela peut provenir d'un retrait intempestif du masque. Ainsi, lorsque les variations temporelles du débit ne sont pas suffisamment marquées au cours d'une période temporelle prédéterminée, c'est-à-dire lorsque les variations temporelles sont inférieures à un seuil de variation prédéterminé, les sources de lumière peuvent être activées selon la séquence d'alerte.

La figure 6B résume les principales étapes du mode de réalisation précédemment décrit :
Etape 100 : mesure du débit d'air par le capteur de débit et/ou de la pression d'air par le capteur de pression, à un instant de mesure ;
Etape 110 : réitération de l'étape 100 ;
Etape 140 : comparaison de chaque débit mesuré ou de chaque pression mesurée par rapport à une valeur seuil ou à une plage de valeur. Cette étape peut comporter une détermination d'une variation de différents débits mesurés et une comparaison de la variation avec un seuil de variation.
Etape 150 : en fonction de la comparaison, activation d'une séquence d'alerte.

Dans les exemples décrits ci-dessus et illustrés sur les figures 1 à 4, l'enceinte 10 présente une forme sensiblement cylindrique. D'autres formes sont naturellement envisageables, par exemple une forme hémisphérique ou une forme conique.

## Revendications

1. Dispositif de ventilation respiratoire (1), destiné à envoyer un flux d'air, généré par un ventilateur, dans un conduit (2), le conduit s'étendant entre le dispositif et un masque respiratoire (3), destiné à être porté par un utilisateur, le dispositif comportant :
- une entrée d'air (10i), destinée à admettre l'air dans le dispositif (1);
- un ventilateur ;
- une sortie d'air (10o), la sortie d'air étant configurée pour être raccordée au conduit (2); de telle sorte que lorsque le ventilateur fonctionne, l'air s'écoule, à travers le dispositif, depuis l'entrée d'air successivement vers le ventilateur puis vers la sortie d'air ;
- un capteur de débit (25), mesurant un débit d'air circulant à travers le dispositif, et/ou un capteur de pression (26) mesurant une pression entre le ventilateur et la sortie d'air;
- une unité de commande (18), reliée au capteur de débit (25) et/ou au capteur de pression (26) ;
- une enceinte (10), définissant l'entrée d'air (10i) et la sortie d'air (10o), l'enceinte renfermant le ventilateur,
- l'enceinte s'étendant, autour d'un axe longitudinal (Z), entre une base (10_{inf}) et un sommet (10ₛᵤₚ);
- l'enceinte comportant une paroi latérale (11, 12, 13) reliant la base et le sommet, la paroi latérale s'étendant autour de l'axe longitudinal (Z) ;
- le dispositif étant **caractérisé en ce que**
- l'enceinte comprenant au moins un support (40, 40ₐ, 40_{b}), formant une courbe, et s'étendant à l'intérieur de l'enceinte, autour de l'axe longitudinal (Z), le support étant disposé en regard de la paroi latérale, le support portant une pluralité de sources de lumière (41), chaque source de lumière étant configurée pour émettre une lumière ;
- l'enceinte étant formée d'un matériau translucide et/ou diffusant ; de telle sorte que sous l'effet d'une activation des sources de lumière fixées au support, la lumière, produite par les sources de lumière, diffuse à travers la paroi latérale de l'enceinte.

2. Dispositif selon la revendication 1, dans lequel:
- la paroi latérale s'étend autour de l'axe longitudinal (Z) selon un premier rayon (r₁);
- l'enceinte renferme un réflecteur de lumière (30), le réflecteur s'étendant autour de l'axe longitudinal, selon un deuxième rayon (r₂) inférieur au premier rayon (r₁) ;
- de telle sorte que le réflecteur (30) est apte à réfléchir une lumière produite par les sources de lumière (41) vers l'enceinte (10).

3. Dispositif selon l'une quelconque des revendications 1 à 2, dans lequel les sources de lumière (41) sont configurées pour émettre une lumière en direction du sommet (10ₛᵤₚ) de l'enceinte.

4. Dispositif selon l'une quelconque des revendications précédentes, dans lequel au moins une source de lumière (41), voire chaque source de lumière (41), émet une lumière selon un cône d'émission (Ω) s'étendant autour d'un axe d'émission (Δ), l'axe d'émission de la source de lumière, ou de chaque source de lumière, étant orienté en direction du sommet (10ₛᵤₚ) de l'enceinte.

5. Dispositif selon l'une quelconque des revendications précédentes, dans lequel les sources de lumière (41) sont espacées autour de l'axe longitudinal (Z).

6. Dispositif selon l'une quelconque des revendications précédentes, dans lequel au moins une source de lumière (41), voire chaque source de lumière, s'étend entre le support (40) et l'axe longitudinal (Z), de telle sorte que le support (40) est interposé entre au moins une source de lumière, voire chaque source de lumière, et l'enceinte (10).

7. Dispositif selon l'une quelconque des revendications précédentes, dans lequel au moins une source de lumière est pilotée par l'unité de commande, de telle sorte que l'unité de commande (18) est configurée pour activer la source de lumière (41) en fonction du débit d'air mesuré par le capteur de débit (25) et/ou de la pression mesurée par le capteur de pression (26).

8. Dispositif selon l'une quelconque des revendications précédentes, dans lequel l'unité de commande (18) est configurée pour moduler une intensité lumineuse et/ou une couleur de la source de lumière (41) selon une période de modulation (Tᵢ), la période de modulation comportant :
- une augmentation progressive de l'intensité lumineuse suivie d'une diminution progressive de l'intensité lumineuse ;
- et/ou une variation progressive de la couleur entre une couleur de départ et une couleur d'arrivée, suivie d'une variation progressive de la couleur de la lumière émise entre la couleur d'arrivée et la couleur de départ.

9. Dispositif selon la revendication l'une quelconque des revendications 7 à 8, dans lequel l'unité de commande (18) est configurée pour:
- estimer une fréquence respiratoire (T_{Q}) en fonction des débits mesurés du capteur de débit (25) ;
- déterminer la période de modulation (Tᵢ) en fonction de la fréquence respiratoire estimée.

10. Dispositif selon l'une quelconque des revendications 7 à 9, dans lequel l'unité de commande (18) est configurée pour :
- estimer un rythme cardiaque (HR) fonction des débits mesurés par le capteur de débit (25) ;
- déterminer la période de modulation (Tᵢ) en fonction du rythme cardiaque estimé.

11. Dispositif selon l'une quelconque des revendications 7 à 10, dans lequel l'unité de commande (18) est configurée pour activer la source de lumière, selon une séquence d'alerte, lorsque le débit d'air est en dehors d'une plage de débit prédéterminée.

12. Dispositif selon l'une quelconque des revendications 7 à 11, dans lequel l'unité de commande (18) est configurée pour déterminer une variation temporelle des débits mesurés par le capteur de débit, en différents instants successifs, et pour activer la source de lumière, selon une séquence d'alerte, lorsque la variation temporelle est inférieure à un seuil de variation prédéfini.

13. Dispositif selon l'une quelconque des revendications 7 à 12, dans lequel l'unité de commande (18) est configurée pour activer la source de lumière, selon une séquence d'alerte, lorsque la pression d'air est en dehors d'une plage de pression prédéterminée.

14. Dispositif selon l'une quelconque des revendications précédentes, dans lequel l'enceinte comporte une horloge (19'), reliée à l'unité de commande (18), de telle sorte que l'unité de commande est configurée pour moduler une intensité lumineuse des sources de lumière en fonction de l'horloge.

15. Procédé de commande d'une source de lumière d'un dispositif selon l'une quelconque des revendications précédentes, comportant les étapes suivantes :
a) mesure de débits à l'aide du capteur de débit (25) et/ou de pressions à l'aide du capteur de pression (26) respectivement à différents instants de mesure ;
b) en fonction des débits et/ou de pressions mesurés, activation de la source de lumière par l'unité de commande (18).

16. Procédé selon la revendication 15, dans lequel lors de l'étape b), l'activation de la source de lumière est configurée pour moduler une intensité lumineuse et/ou une couleur de la source de lumière (41) selon une période de modulation (Tᵢ), la période de modulation comportant:
- une augmentation progressive de l'intensité lumineuse suivie d'une diminution progressive de l'intensité lumineuse ;
- et/ou une variation progressive de la couleur entre une couleur de départ et une couleur d'arrivée, suivie d'une variation progressive de la couleur de la lumière émise entre la couleur d'arrivée et la couleur de départ.

17. Procédé selon la revendication 15, dans lequel l'étape b) comporte une activation de la source de lumière selon une séquence d'alerte lorsque :
- un débit d'air mesuré lors de l'étape a) est en dehors d'une plage de débit prédéterminée;
- et/ou une variation du débit d'air, durant une plage temporelle déterminée, est inférieure à un seuil de variation ;
- et/ou une pression d'air mesurée lors de l'étape a) est en dehors d'une plage de pression prédéterminée.

## Patentansprüche

1. Lungenventilationsvorrichtung (1), die für das Schicken eines durch einen Ventilator erzeugten Luftstroms durch eine Leitung (2) bestimmt ist, wobei die Leitung sich zwischen der Vorrichtung und einer Atemmaske (3), die für das Tragen durch einen Benutzer bestimmt ist, erstreckt, wobei die Vorrichtung umfasst:
- einen Lufteinlass (10i), der zum Einlassen der Luft in die Vorrichtung (1) bestimmt ist;
- einen Ventilator;
- einen Luftauslass (10o), wobei der Luftauslass zum Anschließen an die Leitung (2) konfiguriert ist;
so dass, wenn der Ventilator läuft, die Luft durch die Vorrichtung vom Lufteinlass weiter zum Ventilator und dann zum Luftauslass strömt;
- einen Strömungssensor (25), der einen Luftdurchsatz misst, der durch die Vorrichtung zirkuliert, und/oder einen
Drucksensor (26), der einen Druck zwischen dem Ventilator und dem Luftauslass misst;
- eine Steuereinheit (18), die mit dem Strömungssensor (25) und/oder mit dem Drucksensor (26) verbunden ist;
- ein Gehäuse (10), das den Lufteinlass (10i) und den Luftauslass (10o) definiert, wobei das Gehäuse den Ventilator umschließt,
- wobei das Gehäuse sich um eine Längsachse (Z) zwischen einer Basis (10_{inf}) und einem Scheitel (10ₛᵤₚ) erstreckt;
- wobei das Gehäuse eine Seitenwand (11, 12, 13) umfasst, die die Basis und den Scheitel verbindet, wobei die Seitenwand sich um die Längsachse (Z) erstreckt;
- wobei die Vorrichtung **dadurch gekennzeichnet ist, dass**
- das Gehäuse mindestens eine Halterung (40, 40ₐ, 40_{b}) umfasst, die eine Krümmung bildet und sich im Innern des Gehäuses um die Längsachse (Z) erstreckt, wobei die Halterung in Bezug auf die Seitenwand angeordnet ist und die Halterung mehrere Lichtquellen (41) trägt, wobei jede Lichtquelle dazu konfiguriert ist, ein Licht zu emittieren;
- das Gehäuse aus einem durchscheinenden und/oder lichtstreuenden Material gebildet ist;
so dass unter der Wirkung einer Aktivierung der Lichtquellen, die an der Halterung befestigt sind, das durch die Lichtquellen erzeugte Licht durch die Seitenwand des Gehäuses streut.

2. Vorrichtung nach Anspruch 1, wobei
- die Seitenwand sich in einem ersten Radius (r₁) um die Längsachse (Z) erstreckt;
- das Gehäuse einen Lichtreflektor (30) einschließt, wobei der Reflektor sich in einem zweiten Radius (r₂), der kleiner als der erste Radius (r₁) ist, um die Längsachse erstreckt;
- so dass der Reflektor (30) geeignet ist, ein durch die Lichtquellen (41) erzeugtes Licht zum Gehäuse (10) zu reflektieren.

3. Vorrichtung nach einem der Ansprüche 1 oder 2, wobei die Lichtquellen (41) dazu konfiguriert sind, ein Licht in Richtung des Scheitels (10ₛᵤₚ) des Gehäuses zu emittieren.

4. Vorrichtung nach einem der vorhergehenden Ansprüche, wobei mindestens eine Lichtquelle (41), sogar jede Lichtquelle (41) ein Licht entsprechend einem Emissionskonus (Ω) emittiert, der sich um eine Emissionsachse (Δ) erstreckt, wobei die Emissionsachse der Lichtquelle oder jeder Lichtquelle in Richtung des Scheitels (10ₛᵤₚ) des Gehäuses ausgerichtet ist.

5. Vorrichtung nach einem der vorhergehenden Ansprüche, wobei die Lichtquellen (41) um die Längsachse (Z) beabstandet sind.

6. Vorrichtung nach einem der vorhergehenden Ansprüche, wobei mindestens eine Lichtquelle (41), sogar jede Lichtquelle, sich zwischen der Halterung (40) und der Längsachse (Z) erstreckt, so dass die Halterung (40) zwischen mindestens einer Lichtquelle, sogar jeder Lichtquelle, und dem Gehäuse (10) angeordnet ist.

7. Vorrichtung nach einem der vorhergehenden Ansprüche, wobei mindestens eine Lichtquelle durch die Steuereinheit gesteuert wird, so dass die Steuereinheit (18) dazu konfiguriert ist, die Lichtquelle (41) in Abhängigkeit vom Luftdurchsatz, der durch den Strömungssensor (25) gemessen wird, und/oder vom Druck, der durch den Drucksensor (26) gemessen wird, zu aktivieren.

8. Vorrichtung nach einem der vorhergehenden Ansprüche, wobei die Steuereinheit (18) dazu konfiguriert ist, ist, eine Lichtstärke und/oder eine Farbe der Lichtquelle (41) entsprechend einer Modulationsperiode (Tᵢ) zu modulieren, wobei die Modulationsperiode umfasst:
- eine progressive Steigerung der Lichtstärke, gefolgt von einer progressiven Verringerung der Lichtstärke;
- und/oder eine progressive Variation der Farbe zwischen einer Ausgangsfarbe und einer Zielfarbe, gefolgt von einer progressiven Variation der Farbe des emittierten Lichts zwischen der Zielfarbe und der Ausgangsfarbe.

9. Vorrichtung nach Anspruch einem der Ansprüche 7 bis 8, wobei die Steuereinheit (18) konfiguriert ist zum:
- Schätzen einer Atemfrequenz (T_{Q}) in Abhängigkeit von den Durchsätzen, die vom Strömungssensor (25) gemessen werden;
- Bestimmen der Modulationsperiode (Tᵢ) in Abhängigkeit von der geschätzten Atemfrequenz.

10. Vorrichtung nach einem der Ansprüche 7 bis 9, wobei die Steuereinheit (18) konfiguriert ist zum:
- Schätzen eines Herzrhythmus (HR) in Abhängigkeit von den Durchsätzen, die vom Strömungssensor (25) gemessen werden;
- Bestimmen der Modulationsperiode (Tᵢ) in Abhängigkeit vom geschätzten Herzrhythmus.

11. Vorrichtung nach einem der Ansprüche 7 bis 10, wobei die Steuereinheit (18) dazu konfiguriert ist, die Lichtquelle entsprechend einer Warnsequenz zu aktivieren, wenn der Luftdurchsatz außerhalb eines vorbestimmten Durchsatzbereichs liegt.

12. Vorrichtung nach einem der Ansprüche 7 bis 11, wobei die Steuereinheit (18) dazu konfiguriert ist, eine zeitliche Variation der vom Strömungssensor gemessenen Durchsätze zu verschiedenen aufeinanderfolgenden Zeitpunkten zu bestimmen, um die Lichtquelle entsprechend einer Warnsequenz zu aktivieren, wenn die zeitliche Variation unter einem vorbestimmten Variationsschwellenwert liegt.

13. Vorrichtung nach einem der Ansprüche 7 bis 12, wobei die Steuereinheit (18) dazu konfiguriert ist, die Lichtquelle entsprechend einer Warnsequenz zu aktivieren, wenn der Luftdruck außerhalb eines vorbestimmten Druckbereichs liegt.

14. Vorrichtung nach einem der vorhergehenden Ansprüche, wobei das Gehäuse einen Taktgeber (19') umfasst, der mit der Steuereinheit (18) verbunden ist, so dass die Steuereinheit dazu konfiguriert ist, eine Lichtstärke der Lichtquellen in Abhängigkeit vom Taktgeber zu modulieren.

15. Verfahren zur Steuerung einer Lichtquelle einer Vorrichtung nach einem der vorhergehenden Ansprüche, das die folgenden Schritte umfasst:
a) Messen von Durchsätzen mithilfe des Strömungssensors (25) bzw. und/oder von Drücken mithilfe des Drucksensors (26) zu verschiedenen Messzeitpunkten;
b) in Abhängigkeit von den gemessenen Durchsätzen und/oder Drücken Aktivieren der Lichtquelle durch die Steuereinheit (18).

16. Verfahren nach Anspruch 15, wobei in Schritt b) das Aktivieren der Lichtquelle dazu konfiguriert ist, eine Lichtstärke und/oder eine Farbe der Lichtquelle (41) entsprechend einer Modulationsperiode (Tᵢ) zu modulieren, wobei die Modulationsperiode umfasst:
- eine progressive Steigerung der Lichtstärke, gefolgt von einer progressiven Verringerung der Lichtstärke;
- und/oder eine progressive Variation der Farbe zwischen einer Ausgangsfarbe und einer Zielfarbe, gefolgt von einer progressiven Variation der Farbe des emittierten Lichts zwischen der Zielfarbe und der Ausgangsfarbe.

17. Verfahren nach Anspruch 15, wobei Schritt b) ein Aktivieren der Lichtquelle entsprechend einer Warnsequenz umfasst, wenn:
- wenn der in Schritt a) gemessene Luftdurchsatz außerhalb eines vorbestimmten Durchsatzbereichs liegt;
- und/oder eine Variation des Luftdurchsatzes während eines bestimmten Zeitbereichs unter einem vorbestimmten Variationsschwellenwert liegt;
- und/oder ein in Schritt a) gemessener Luftdruck außerhalb eines vorbestimmten Druckbereichs liegt.

## Claims

1. Respiratory ventilation device (1), intended to send an airflow, generated by a fan, into a duct (2), the duct extending between the device and a respiratory mask (3), intended to be worn by a user, the device comprising:
- an air inlet (10i), intended to admit air into the device (1);
- a fan;
- an air outlet (10o), the air outlet being configured to be connected to the duct (2);
such that when the fan is operating, air flows, through the device, from the air inlet successively to the fan then to the air outlet;
- a flow sensor (25), measuring a flow rate of air flowing through the device, and/or a pressure sensor (26) measuring a pressure between the fan and the air outlet;
- a control unit (18), connected to the flow sensor (25) and/or to the pressure sensor (26);
- an enclosure (10), defining the air inlet (10i) and the air outlet (10o), the enclosure containing the fan,
- the enclosure extending, around a longitudinal axis (Z), between a base (10_{inf}) and a top (10ₛᵤₚ);
- the enclosure comprising a side wall (11, 12, 13) connecting the base and the top, the side wall extending around the longitudinal axis (Z);
the device being **characterized in that**
- the enclosure comprises at least one holder (40, 40ₐ, 40_{b}), forming a curve, and extending inside the enclosure, around the longitudinal axis (Z), the holder being placed facing the side wall, the holder bearing a plurality of light sources (41), each light source being configured to emit a light;
- the enclosure is formed from a translucent and/or scattering material;
such that under the effect of an activation of the light sources fastened to the holder, the light, produced by the light sources, diffuses through the side wall of the enclosure.

2. Device according to Claim 1, wherein:
- the side wall extends around the longitudinal axis (Z) at a first radius (r₁);
- the enclosure contains a reflector of light (30), the reflector extending around the longitudinal axis, at a second radius (r₂) less than the first radius (r₁);
- such that the reflector (30) is able to reflect a light produced by the light sources (41) toward the enclosure (10).

3. Device according to claim 1 or claim 2, wherein the light sources (41) are configured to emit a light in the direction of the top (10ₛᵤₚ) of the enclosure.

4. Device according to any one of the preceding claims, wherein at least one light source (41), or even each light source (41), emits a light in an emission cone (Ω) extending around an emission axis (Δ), the emission axis of the light source, or of each light source, being oriented in the direction of the top (10ₛᵤₚ) of the enclosure.

5. Device according to any one of the preceding claims, wherein the light sources (41) are spaced apart around the longitudinal axis (Z).

6. Device according to any one of the preceding claims, wherein at least one light source (41), or even each light source, lies between the holder (40) and the longitudinal axis (Z), such that the holder (40) is interposed between at least one light source, or even each light source, and the enclosure (10).

7. Device according to any one of the preceding claims, wherein at least one light source is driven by the control unit, such that the control unit (18) is configured to activate the light source (41) depending on the flow rate of air measured by the flow sensor (25) and/or on the pressure measured by the pressure sensor (26).

8. Device according to any one of the preceding claims, wherein the control unit (18) is configured to modulate a light intensity and/or a colour of the light source (41) with a modulation period (Tᵢ), the modulation period comprising:
- a gradual increase in light intensity followed by a gradual decrease in light intensity;
- and/or a gradual variation in colour between a start colour and an end colour, followed by a gradual variation in the colour of the emitted light between the end colour and the start colour.

9. Device according to claim any one of Claims 7 to 8, wherein the control unit (18) is configured to:
- estimate a respiratory rate (T_{Q}) depending on the measured flow rates of the flow sensor (25);
- define the modulation period (Tᵢ) depending on the estimated respiratory rate.

10. Device according to any one of Claims 7 to 9, wherein the control unit (18) is configured to:
- estimate a heart rate (HR) depending on the flow rates measured by the flow sensor (25);
- define the modulation period (Tᵢ) depending on the estimated heart rate.

11. Device according to any one of Claims 7 to 10, wherein the control unit (18) is configured to activate the light source, in an alarm sequence, when the air flow rate is outside of a predetermined flow-rate range.

12. Device according to any one of Claims 7 to 11, wherein the control unit (18) is configured to determine a variation as a function of time in the flow rates measured by the flow sensor, at various successive times, and to activate the light source, in an alarm sequence, when the variation as a function of time is less than a predefined variation threshold.

13. Device according to any one of Claims 7 to 12, wherein the control unit (18) is configured to activate the light source, in an alarm sequence, when the air pressure is outside of a predetermined pressure range.

14. Device according to any one of the preceding claims, wherein the enclosure comprises a clock (19'), connected to the control unit (18), such that the control unit is configured to modulate a light intensity of the light sources depending on the clock.

15. Method for controlling a light source of a device according to any one of the preceding claims, comprising the following steps:
a) measurement of flow rates using the flow sensor (25) and/or of pressures using the pressure sensor (26) at various measurement times, respectively;
b) depending on the measured flow rates and/or pressures, activation of the light source with the control unit (18).

16. Method according to Claim 15, wherein, in step b), the activation of the light source is configured to modulate a light intensity and/or a colour of the light source (41) with a modulation period (Tᵢ), the modulation period comprising:
- a gradual increase in light intensity followed by a gradual decrease in light intensity;
- and/or a gradual variation in colour between a start colour and an end colour, followed by a gradual variation in the colour of the emitted light between the end colour and the start colour.

17. Method according to Claim 15, wherein step b) comprises an activation of the light source in an alarm sequence when:
- an air flow rate measured in step a) is outside of a predetermined flow-rate range;
- and/or a variation in the air flow rate, during a determined time range, is less than a variation threshold;
- and/or an air pressure measured in step a) is outside of a predetermined pressure range.
